(19) Europäisches Patentamt
European Patent Office
Office européen des brevets

(11) EP 4 736 829 A1

(12) EUROPEAN PATENT APPLICATION
published in accordance with Art. 153(4) EPC

(43) Date of publication:
06.05.2026 Bulletin 2026/19

(51) International Patent Classification (IPC):
A61G 10/02 (2006.01)    A61G 10/00 (2006.01)
A61B 5/03 (2006.01)    H04R 1/08 (2006.01)
H04R 1/10 (2026.01)    H04R 1/32 (2006.01)

(21) Application number: 24922666.3

(52) Cooperative Patent Classification (CPC):
A61B 5/03; A61G 10/00; A61G 10/02; H04R 1/08;
H04R 1/10; H04R 1/32

(22) Date of filing: 26.08.2024

(86) International application number:
PCT/KR2024/012675

(87) International publication number:
WO 2026/049070 (05.03.2026 Gazette 2026/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Ibex Medical Systems Co., Ltd.
Wonju-si, Gangwon-do 26348 (KR)

(72) Inventor: The designation of the inventor has not
yet been filed

(74) Representative: Manitz Finsterwald
Patent- und Rechtsanwaltspartnerschaft mbB
Martin-Greif-Straße 1
80336 München (DE)

(54) **AUTOMATIC PRESSURE CONTROL SYSTEM AND METHOD FOR HYPERBARIC OXYGEN CHAMBER FOR DETECTING PRESSURE EQUILIBRIUM STATE BETWEEN OUTER EAR AND MIDDLE EAR**

(57) According to the present disclosure, in an automatic pressure control system of a hyperbaric oxygen chamber for detecting pressure equalization between the external ear and the middle ear, at least one processor may be configured to calculate an admittance of the eardrum based on an energy level of an output wave output by a speaker and an energy level of a reflected wave received by a microphone, determine whether the pressure equalization has occurred based on changes in the calculated admittance, and control an air feed valve based on the determination of whether the pressure equalization has occurred.

*FIG. 1*

**Description**

## TECHNICAL FIELD

The present disclosure relates to an automatic pressure control system and method of a hyperbaric oxygen chamber for detecting pressure equalization between the external ear and the middle ear.

## BACKGROUND

Hyperbaric oxygen therapy is a medical treatment that involves supplying nearly pure oxygen to a patient in a pressurized environment at a pressure more than twice that of the atmospheric pressure using a hyperbaric oxygen chamber. It is mainly used for conditions such as decompression sickness, air embolism, gas poisoning, wound healing, and burn treatment.

In a conventional automatic pressure control system of a hyperbaric oxygen chamber, the pressure is controlled based on a treatment table without considering the patient's condition. However, during hyperbaric oxygen therapy, the pressure difference between the external ear and the middle ear in the pressurized environment can cause middle ear barotrauma. Depending on the severity, middle ear barotrauma may result in mild pain, congestion, and bleeding, and in severe cases, may cause eardrum rupture.

To prevent the occurrence of middle ear barotrauma, the use of nasal sprays and extremely slow pressurization have been implemented. However, these methods cannot completely prevent the occurrence of middle ear barotrauma. A major issue is that the occurrence of middle ear barotrauma during treatment must be determined by the medical personnel based only on the patient's subjective feedback obtained through conversation.

Therefore, a patient-customized treatment system is needed that can objectively determine the occurrence of middle ear barotrauma during hyperbaric oxygen therapy and can adjust the pressurization level according to the patient's condition.

## DISCLOSURE OF THE INVENTION

## PROBLEMS TO BE SOLVED BY THE INVENTION

The present disclosure is conceived to objectively determine the pressure difference between the external ear and the middle ear of a patient undergoing hyperbaric oxygen therapy.

Also, the present disclosure is conceived to automatically control the pressure of a hyperbaric oxygen chamber to protect a patient undergoing hyperbaric oxygen therapy from middle ear barotrauma.

The problems to be solved by the present disclosure are not limited to the above-described problems. There may be other problems to be solved by the present disclosure.

## MEANS FOR SOLVING THE PROBLEMS

An automatic pressure control system for a hyperbaric oxygen chamber for detecting pressure equalization between the external ear and the middle ear may comprise, a chamber having an internal space in which a patient can be positioned, a probe configured to be inserted into the patient's ear and having a speaker configured to output sound waves and a microphone configured to receive sound waves, an MCU module electrically connected to the probe, an air feed valve configured to open or close an air feed line connected to the chamber, an air exhaust valve configured to open or close an air exhaust line connected to the chamber, and at least one processor. Wherein the MCU module may be configured to transmit an output wave through the speaker and receives a reflected wave of the output wave reflected from the patient's eardrum through the microphone. Wherein the at least one processor may be configured to, calculate an admittance of the eardrum based on an energy level of the output wave output by the speaker and an energy level of the reflected wave received by the microphone, determine whether the pressure equalization has occurred based on changes in the calculated admittance, and control the air feed valve based on the determination of whether the pressure equalization has occurred.

An automatic pressure control method for a hyperbaric oxygen chamber for detecting pressure equalization between the external ear and the middle ear may comprise, transmitting an output wave through a speaker and receiving a reflected wave of the output wave reflected from a patient's eardrum through a microphone, wherein the speaker and the microphone included in a probe configured to be inserted into the patient's ear, calculating an admittance of the eardrum based on an energy level of the output wave output by the speaker and an energy level of the reflected wave received by the microphone, determining whether the pressure equalization has occurred based on changes in the calculated admittance, and controlling an air feed valve, which is configured to open or close an air feed line connected to a chamber, based on the determination of whether the pressure equalization has occurred.

Effects of the invention

According to an embodiment of the present disclosure, the pressure difference between the external ear and the middle ear can be objectively determined by detecting the condition of the patient's eardrum.

Accordingly, it is possible to prevent the occurrence of middle ear barotrauma in the patient, ensure safety, and improve the efficiency of pressure control in the hyperbaric oxygen chamber.

**BRIEF DESCRIPTION OF THE DRAWINGS**

**FIG. 1** is a schematic block diagram illustrating an automatic pressure control system of a hyperbaric oxygen chamber according to an embodiment of the present disclosure.

**FIG. 2** is a signal flowchart illustrating the automatic pressure control system of the hyperbaric oxygen chamber according to an embodiment of the present disclosure.

**FIG. 3** illustrates a probe according to an embodiment of the present disclosure.

**FIG. 4** is a view illustrating a state in which the probe is inserted into a patient's ear according to an embodiment of the present disclosure.

**FIG. 5** is a block diagram illustrating an MCU module according to an embodiment of the present disclosure.

**FIG. 6** is a circuit diagram of a speaker driving circuit according to an embodiment of the present disclosure.

**FIG. 7** is a circuit diagram of a microphone driving circuit according to an embodiment of the present disclosure.

**FIG. 8A** to **FIG. 8C** show user interface screens of an automatic pressure control program according to an embodiment of the present disclosure.

**FIG. 9** is a graph showing the result of admittance measurements when the Valsalva maneuver is performed.

**FIG. 10** is a graph showing a pattern of change in admittance when the Valsalva maneuver is performed.

**FIG. 11** is a graph showing the result of admittance measurements according to swallowing.

**FIG. 12** is a graph showing a pattern of change in admittance according to swallowing.

**FIG. 13** is a flowchart showing an automatic pressure control method of a hyperbaric oxygen chamber for detecting pressure equalization between the external ear and the middle ear according to an embodiment of the present disclosure.

**BEST MODE FOR CARRYING OUT THE INVENTION**

Hereinafter, embodiments of the present disclosure will be described in detail with reference to the accompanying drawings to enable those skilled in the art to easily practice the present disclosure. However, the present disclosure may be implemented in various different forms and is not limited to the embodiments described herein. Parts unrelated to the description are omitted in the drawings to clearly explain the present disclosure, and like reference numerals designate like elements throughout the specification.

Throughout the present specification, when a member is described as being positioned "on" another member, this includes not only cases where the member is in direct contact with the other member but also cases where an intervening member exists between them.

Throughout the present specification, when a part is described as "comprising" a component, this means it may further include other components unless explicitly stated otherwise, rather than excluding them.

Throughout the present specification, terms of degree such as "about" or "substantially" are used to account for inherent manufacturing and material tolerances associated with the stated meaning, or to prevent unscrupulous infringers from unfairly exploiting disclosures mentioning precise or absolute numerical values.

Throughout the present specification, the term "step of ~ing" or "step of ~" does not mean "step for ~."

Throughout the present specification, the phrase "combination(s) thereof" in a Markush-type expression refers to one or more mixtures or combinations selected from the group consisting of the listed components, meaning the inclusion of at least one selected from said group.

Throughout the present specification, the recitation "A and/or B" means "A, B, or both A and B."

Hereinafter, implementations and embodiments of the present disclosure will be described in detail with reference to the attached drawings. However, the present disclosure is not limited to these implementations, embodiments, or drawings.

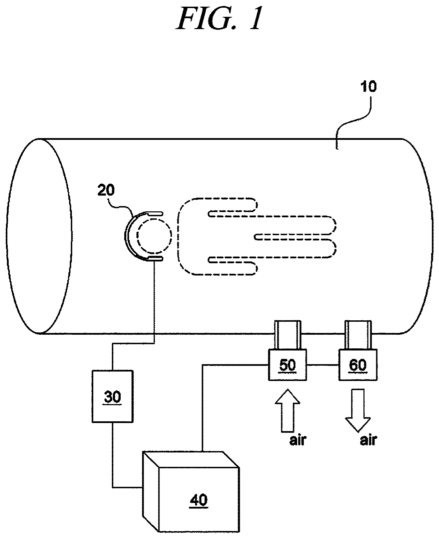

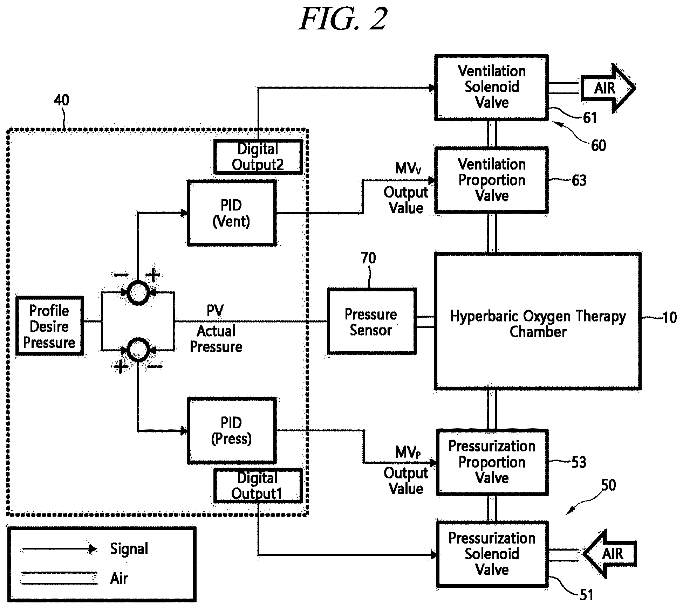

**FIG. 1** is a schematic block diagram illustrating an automatic pressure control system of a hyperbaric oxygen chamber according to an embodiment of the present disclosure. **FIG.** 2 is a signal flowchart illustrating the automatic pressure control system of the hyperbaric oxygen chamber according to an embodiment of the present disclosure.

Referring to **FIG. 1** and **FIG. 2,** an automatic pressure control system of a hyperbaric oxygen chamber according to an embodiment of the present disclosure includes a chamber 10, a probe 20, an MCU module 30, an air feed valve 50, an air exhaust valve 60, and/or a processor 40.

According to an embodiment, the chamber 10 may be configured to allow a patient to enter and receive hyperbaric oxygen therapy. To this end, the chamber 10 may be formed as a sealed container structure with an internal space in which the patient can be positioned, and may be provided with an entrance.

According to an embodiment, the chamber 10 may have a cylindrical shape. Therefore, the pressure resistance to withstand the pressure difference between the inside and outside of the chamber 10 can be improved. In an embodiment, at least one of a bed or a chair may be provided inside the chamber 10.

According to an embodiment, the probe 20 may be configured to emit sound waves and receive the reflected sound waves in order to measure the condition of the eardrum. In an embodiment, the probe 20 may be mounted on the patient's ear and may emit sound waves toward the patient's eardrum and receive the sound waves reflected from the eardrum to measure their intensity.

According to an embodiment, the MCU module 30 may be electrically connected to the probe 20 and may be configured to control the probe 20 to emit sound waves and receive the reflected sound waves from the probe 20.

According to an embodiment, the processor 40 may estimate the condition of the eardrum based on the intensity of the sound waves received from the MCU module 30. In an embodiment, the processor 40 may generate a control signal for adjusting the internal pressure of the chamber 10 based on the estimated condition of the eardrum.

According to an embodiment, the processor 40 may be implemented using one or more general-purpose or special-purpose computers. In an embodiment, the processor 40 may execute an operating system (OS) and one or more software applications running on the OS. Further, the processor 40 may access, store, manipulate, process, and generate data in response to the execution of software.

According to an embodiment, the air feed valve 50 may be configured to open or close an air feed line connected to the chamber 10 in response to a control signal from the processor 40. In an embodiment, when the air feed valve 50 is opened, air may be introduced into the chamber 10 and the internal pressure of the chamber 10 may be increased.

According to an embodiment, the air exhaust valve 60 may be configured to open or close an air exhaust line connected to the chamber 10 in response to a control signal from the processor 40. In an embodiment, when the air exhaust valve 60 is opened, air may be discharged from the chamber 10 to the outside and the internal pressure of the chamber 10 may be decreased.

According to an embodiment, the automatic pressure control system of the hyperbaric oxygen chamber may control the pressure through a PID control. In an embodiment, when the treatment begins, the processor 40 may open an air feed solenoid valve 51 and an air exhaust solenoid valve 61.

According to an embodiment, the processor 40 may present a pressure value from a treatment table as a set point SP, obtain an internal pressure value of the chamber 10 via a pressure sensor 70, present the internal pressure value as a process variable PV, and obtain a manipulate variable value MV through a PID operation to control an air feed proportion valve 53 and/or an air exhaust proportion valve 63.

During a pressurization process in hyperbaric oxygen therapy using the chamber 10, barotrauma may occur due to pressure differences between the inside and outside of cells in organs within the body. Barotrauma is derived from a combination of "baros", meaning pressure, and "trauma", meaning injury. Barotrauma that occurs during hyperbaric oxygen therapy may include external auditory canal barotrauma, middle ear barotrauma, inner ear barotrauma, sinus barotrauma, lung barotrauma, and teeth barotrauma. Among these, the most frequently occurring type is middle ear barotrauma, which results from a failure to equalize the pressure between the external ear and the middle ear. If middle ear barotrauma occurs, the patient may experience symptoms ranging from mild pain to hearing loss and bleeding, and in severe cases, eardrum rupture. Middle ear barotrauma can be prevented by opening the Eustachian tube and equalizing the pressure between the external ear and the middle ear. Methods for achieving this pressure equalization include the Toynbee maneuver, the Valsalva maneuver, the Frenzel maneuver, the Lowry technique, the Edmonds technique, swallowing, and yawning.

To prevent the occurrence of middle ear barotrauma during hyperbaric oxygen therapy, the use of nasal sprays and training for breathing maneuver for pressure equalization have been implemented. However, the determination of middle ear barotrauma during treatment relies on communication between the patient and the medical personnel. As a result, even with appropriate preventive measures, the risk of middle ear barotrauma remains. Therefore, there is a need for a method to objectively determine the occurrence of middle ear barotrauma in real time by directly monitoring the condition of the eardrum during hyperbaric oxygen therapy, rather than relying on the patient's subjective sensations.

According to an embodiment of the present disclosure, the automatic pressure control system and method of a hyperbaric oxygen chamber for detecting pressure equalization between the external ear and the middle ear can objectively determine the occurrence of barotrauma through the probe 20.

In an embodiment, the automatic pressure control system and method may determine the condition of the eardrum by measuring its admittance through the probe 20. Herein, the term "admittance" refers to the inverse of impedance and indicates a measure of how easily vibrations (e.g., sound waves) are transmitted. A higher admittance value indicates that vibrations are more easily transmitted. The unit of admittance is mho.

According to an embodiment, the probe 20 may include a speaker 222 for presenting test sounds to the eardrum and a microphone 223 for receiving sound waves reflected from the eardrum.

In an embodiment, the speaker 222 may output a single sound wave (*e.g.,* 226 Hz), which vibrates air particles in the external ear. The eardrum resonates with the sound wave and absorbs a part of it while the unabsorbed part is reflected and received by the microphone 223. Depending on the pressure in the external ear, the eardrum may flex toward the external ear and the middle ear.

Depending on the degree of flexion, the intensity of the absorbed and reflected sound waves may vary. Herein, the single sound wave output from the speaker 222 is the sound pressure, and the reflected sound wave received by the microphone 223 is the sound velocity. By using the measured sound pressure and sound velocity, the admittance of the eardrum in response to pressure changes can be measured.

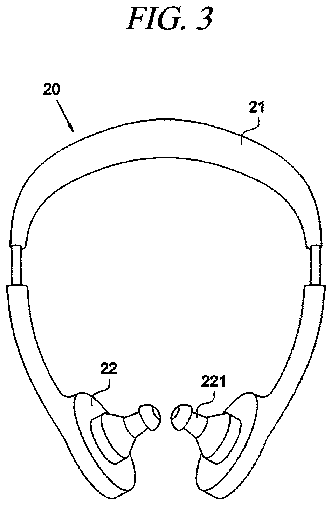

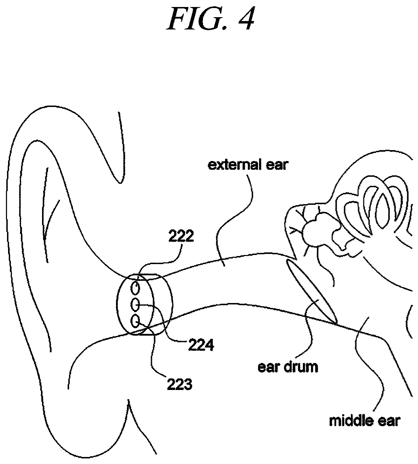

**FIG. 3** illustrates the probe 20 according to an embodiment of the present disclosure. **FIG. 4** is a view illustrating a state in which the probe 20 is inserted into a patient's ear according to an embodiment of the present disclosure.

Referring to **FIG. 3** and **FIG. 4,** the probe 20 according to an embodiment may be provided in the form of a headset. In an embodiment, the probe 20 is provided in the form of a headset including a band 21 and an ear-pad 22. The microphone 223 and the speaker 222 are formed on a protrusion 221 that protrudes from the surface of the headset in contact with the ear and thus can be inserted into the patient's external ear. Since the probe 20 is provided in a form familiar to patients, it becomes easier to guide them on how to wear it.

According to an embodiment, the probe 20 may include the speaker 222 and the microphone 22.

According to an embodiment, the speaker 222 may receive an electrical signal for a test sound from the MCU module 30 and convert it into sound waves to generate the test sound. In an embodiment, the speaker 222 may be inserted into the patient's external ear and emit sound waves toward the eardrum.

In an embodiment, the speaker 222 may have a frequency range of output sound waves from 200 Hz to 55 kHz in which a single sound wave (*e.g.*, 226 Hz) can be output. In an embodiment, the maximum output sound pressure of the speaker 222 may be 127 dB, and the speaker 222 ensures uniform output performance across all frequency bands.

According to an embodiment, the microphone 223 may convert sound waves reflected from the eardrum into electrical signals. In an embodiment, the microphone 223 may be inserted into the patient's external ear with its sound pickup direction oriented toward the eardrum. In an embodiment, the microphone 223 may convert the reflected sound waves from the eardrum into electrical signals and transmit them to the MCU module 30.

In an embodiment, the microphone 223 may have a frequency range of input sound waves from 20 Hz to 10 kHz which may be sufficient to receive a single sound wave (*e.g.,* 226 Hz) reflected from the eardrum.

In an embodiment, the probe 20 may be provided with a passage 224 to equalize the pressure in the external ear to the internal pressure of the chamber 10. Air may flow between the chamber 10 and the external ear through the passage 224, and, thus, the pressure in the external ear can be matched to the internal pressure of the chamber 10.

In an embodiment, the chamber 10 may be provided with the pressure sensor 70. In an embodiment, the processor 40 may obtain the pressure of the external ear by measuring the internal pressure of the chamber 10 via the pressure sensor 70.

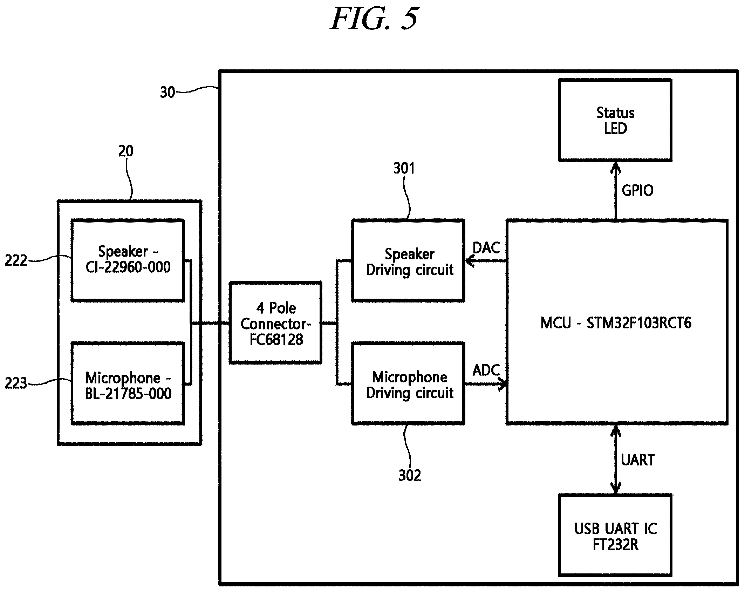

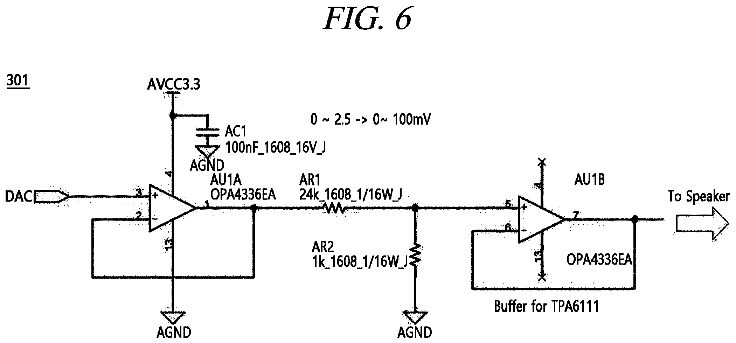

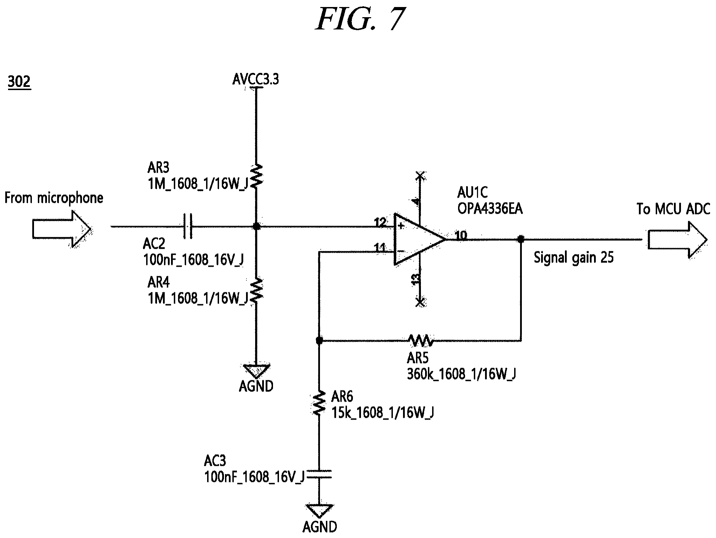

**FIG. 5** is a block diagram illustrating the MCU module 30 according to an embodiment of the present disclosure. **FIG. 6** is a circuit diagram of a speaker driving circuit 301 according to an embodiment of the present disclosure. **FIG. 7** is a circuit diagram of a microphone driving circuit 302 according to an embodiment of the present disclosure.

Referring to **FIG. 5** to **FIG. 7,** the MCU module 30 according to an embodiment may be connected to the probe 20 through a 4-pole AUX cable to exchange signals bidirectionally with the probe 20. In an embodiment, the MCU module 30 may transmit a test sound to the speaker 222 of the probe 20, receive the reflected sound wave as an electrical signal through the microphone 223, and transmit the obtained microphone signal to the processor 40.

According to an embodiment, the MCU module 30 may be an STM32F103RCT6 which uses an ARM-Cortex M3 core as a 32-bit microprocessor for control and computation of the overall system.

According to an embodiment, the MCU module 30 may include an LED unit configured to indicate the system status, a UART unit configured to communicate with the processor 40, and a speaker driving circuit 301 and a microphone driving circuit 302 to drive the speaker 222 and microphone 223, respectively. In an embodiment, an FC6818 as a 4-pole earphone jack may be used to exchange signals with the speaker 222 and microphone 223 of the probe 20.

According to an embodiment, the MCU module 30 may use the speaker driving circuit 301 to control the speaker 222, as shown in **FIG. 6.** For example, the speaker driving circuit 301 may use a digital to analog convert (DAC) of the MCU module 30 to apply a sine wave signal with a frequency of 226 Hz and an amplitude of 0 V to 2.5 V to a DAC input port of the speaker driving circuit 301. Herein, a DAC resolution of the STM32F103RCT6 may be 12-bit.

In an embodiment, since a sine wave with an amplitude of 0 V to 2.5 V has too high a voltage to drive the speaker 222, the voltage may be reduced to 0 V to 100 mV by using an OPAMP voltage divider circuit. The sine wave signal with an attenuated amplitude may vibrate an internal capacitor of the speaker 222 to generate sound pressure.

According to an embodiment, the MCU module 30 may use the microphone driving circuit 302 to control the microphone 223, as shown in **FIG. 7.** In an embodiment, the space velocity input to the microphone 223 may be converted into a voltage and input to the microphone driving circuit 302. For example, the amplitude from the microphone 223 may range from 0 V to 1.3 V and a 226-Hz signal may have a maximum amplitude of 132 mV. Although an input range of an analog to digital converter (ADC) pin of the MCU module 30 is from 0 V to 3.3 V and ADC conversion can be performed without amplification or filtering, the microphone driving circuit 302 may be used to reset a DC offset of the microphone signal to 1.65 V and amplify only the 226-Hz signal by a factor of 25 in order to maximize the resolution. The amplified signal may be input to the ADC pin of the MCU module 30 and then transmitted to the processor 40.

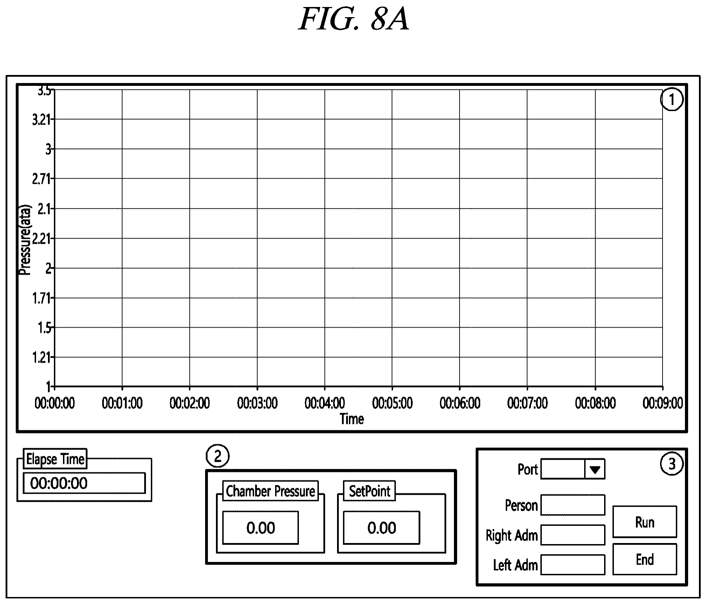

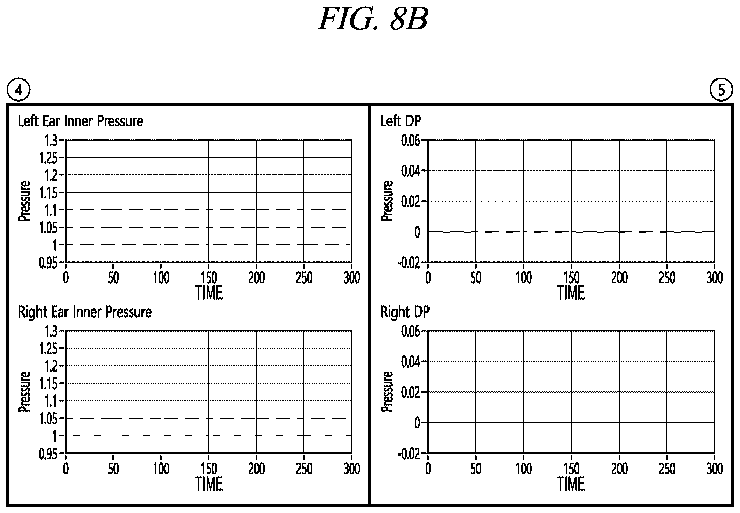

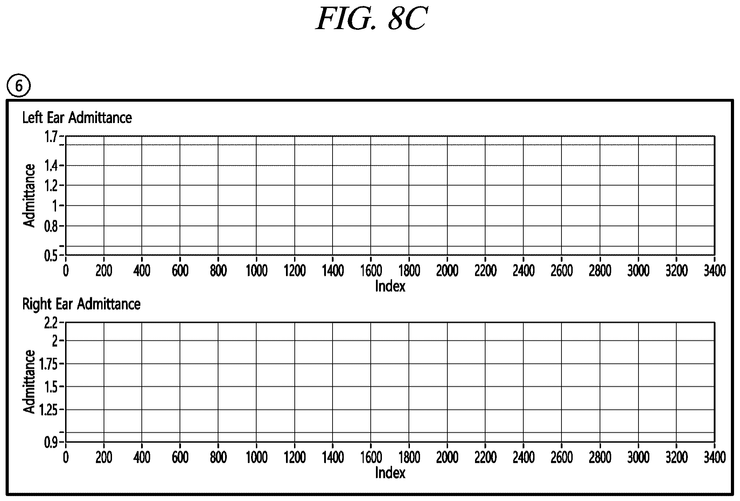

**FIG. 8A** to **FIG. 8C** show user interface screens of an automatic pressure control program according to an embodiment of the present disclosure.

Referring to **FIG. 8A** to **FIG. 8C,** the processor 40 may obtain admittance values of the eardrum from the MCU module 30 and execute an automatic pressure control algorithm based on the obtained values. The processor 40 may be configured with a program developed based on LabView 2010 (National Instruments, USA) to execute the automatic pressure control algorithm.

Specifically, the program is as follows.

①: A graph where the X-axis represents time and the Y-axis represents pressure displays the actual pressure in the chamber 10 during treatment.

②: A portion displays both the actual pressure in the chamber 10 and a pressure set point for the chamber 10 determined by a PID algorithm.

③: A control panel of the processor 40 to start or end the treatment allows the user to set a port for serial communication and input the patient's name.

④: Graphs display the estimated pressures in the left and right middle ears.

⑤: Graphs display the calculated pressure difference between the external ear and the middle ear based on the actual pressure in the chamber 10 and the estimated pressure in the middle ear.

⑥: A real-time graph displays the measured admittance of the eardrum.

According to an embodiment, the automatic pressure control system may execute the automatic pressure control algorithm based on a middle ear pressure estimation algorithm. In an embodiment, the automatic pressure control algorithm (hereinafter, referred to as "Process 1") operates at 1 Hz depending on the speed of the processor 40 while the middle ear pressure estimation algorithm (hereinafter, referred to as "Process 2") operates at a speed of 7,232 Hz (interval: 138.274336 μs) depending on the data transmission rate from the MCU module 30. These two processes operate interactively. The term "Middle ear pressure" refers to the estimated pressure in the middle ear and the term "Chamber Pressure" refers to the actual measured pressure inside the chamber 10, which are used as global variables.

According to an embodiment, the automatic pressure control system of the hyperbaric oxygen chamber may transmit a start command signal from Process 1 to the MCU module 30 at the beginning of treatment. Upon receiving the start command signal, the MCU module 30 may perform Process 2.

In an embodiment, the MCU module 30 may apply a fixed-point digital IIR filter during Process 2 and store filtered data in an array. The designed digital filter is a second-order 226 Hz peak filter with a Q-factor of 50. Through digital filtering, all components except the 226-Hz signal are removed. When the filtered data array reaches 1,024 points, a 1,024-point FFT is executed to compute the power at 226 Hz. The power value is then used to obtain an admittance value of the eardrum.

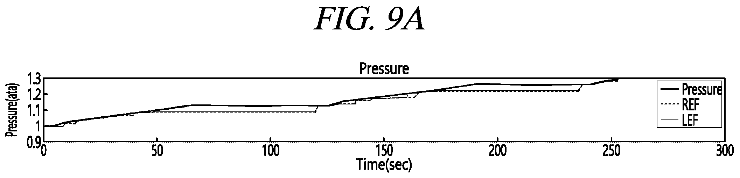

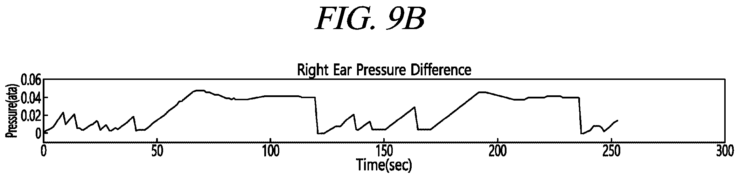

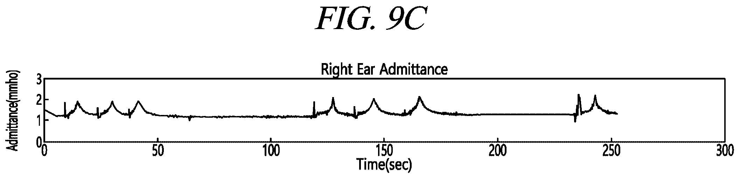

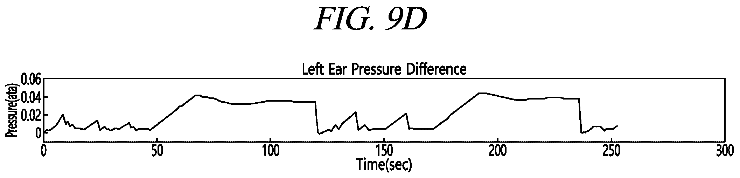

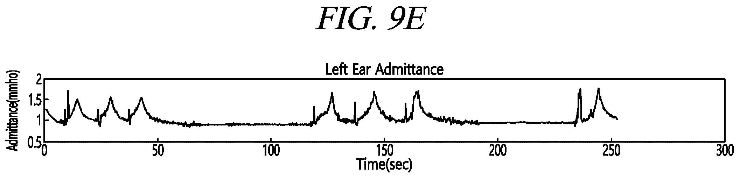

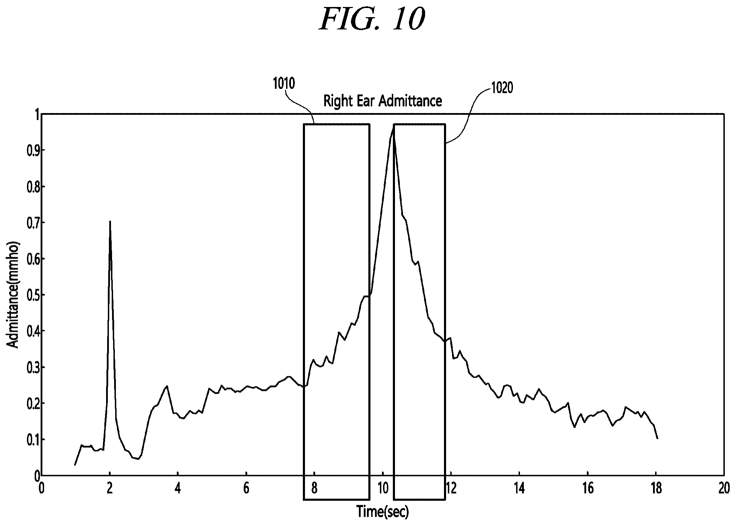

**FIG. 9** is a graph showing the result of admittance measurements when the Valsalva maneuver is performed. **FIG. 10** is a graph showing a pattern of change in admittance when the Valsalva maneuver is performed.

**FIG. 9A** shows the internal pressure of the chamber 10, **FIG. 9B** shows the pressure difference in the right ear, **FIG. 9C** shows the admittance of the right ear, **FIG. 9D** shows the pressure difference in the left ear, and **FIG. 9E** shows the admittance of the left ear. The horizontal axis of each graph represents time.

Referring to **FIG. 9** and **FIG. 10,** the pressurization continues from the point where pressure imbalance begins till the point where the pressure difference between the external ear and the middle ear reaches 0.04 ata. When the pressure difference between the external ear and the middle ear is 0.04 ata, the pressure is observed to be maintained.

When pressure equalization is performed using the Valsalva maneuver, air from the lungs is supplied to the sinuses and the Eustachian tube. The air entering the Eustachian tube causes the eardrum, which flexes toward the middle ear due to external pressure, to flex toward the external ear. In this case, the eardrum passes through a fully recovered state and flexes toward the external ear, but the admittance does not reach its maximum value. This is deemed to be due to data loss caused by rapid changes in the eardrum outpacing the speed at which data can be processed and obtained since the admittance is calculated by overlapping admittance values. However, a momentary peak can be identified as the point of pressure equalization by the algorithm, and the pressurized state can be maintained.

Immediately after the Valsalva maneuver is performed, the admittance of the eardrum reaches its minimum value. Since the admittance value alone cannot determine the direction of eardrum flexion, the system determines the condition of the eardrum as pressure imbalance. However, since pressure equalization has just occurred, the pressure in the chamber 10 continues to increase, which causes the eardrum flexed toward the external area to slowly flex again toward the inner ear and results in the pattern shown in **FIG. 10.** The pressure in the chamber 10 increases at a rate of 0.002 ata per second. Since the time from the Valsalva maneuver to the next pressure equalization point is within 10 seconds, the estimated pressure difference between the external ear and the middle ear remains below 0.04 ata.

As shown in **FIG. 10,** when pressure equalization between the external ear and the inner ear is achieved through the Valsalva maneuver, changes 1010, *i.e.,* a continuous increase, of the admittance prior to the pressure equalization point are followed by changes 1020, *i.e.,* a continuous decrease, of the admittance after the pressure equalization point.

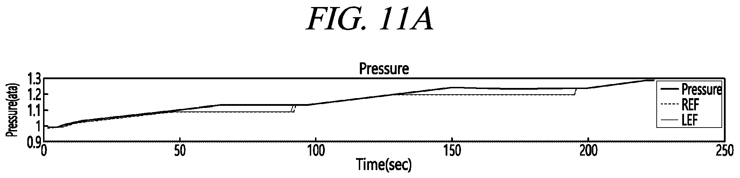

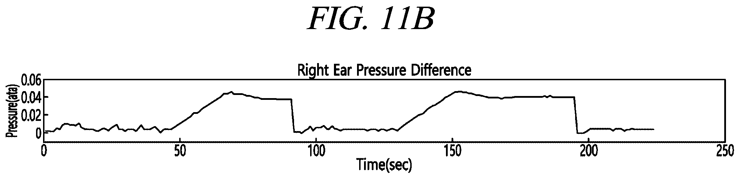

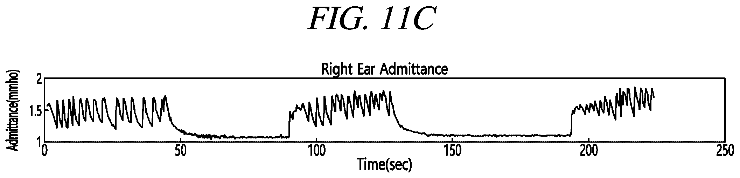

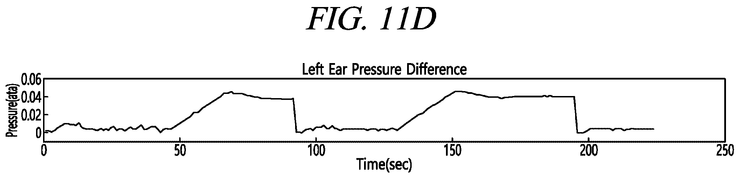

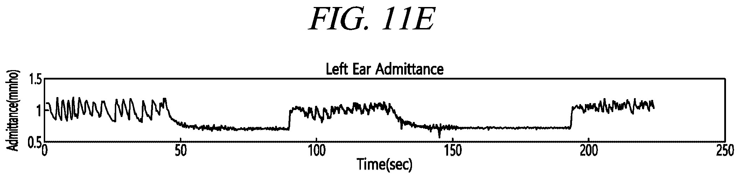

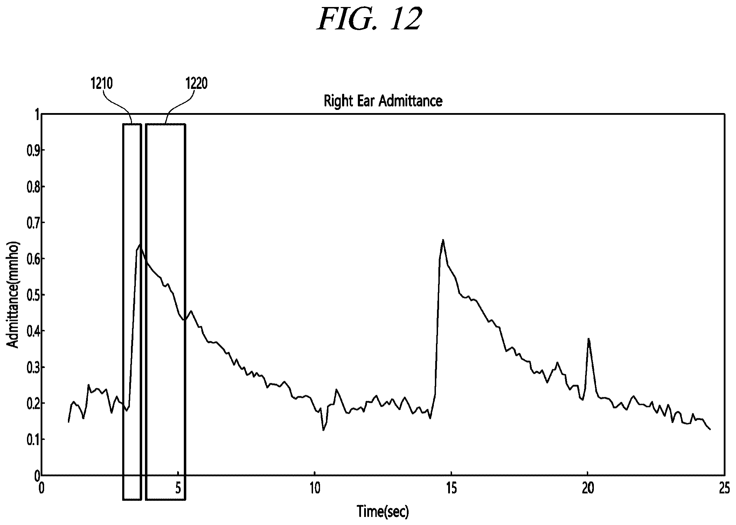

**FIG. 11** is a graph showing the result of admittance measurements according to swallowing. **FIG. 12** is a graph showing a pattern of change in admittance according to swallowing.

**FIG. 11A** shows the internal pressure of the chamber 10, **FIG. 11B** shows the pressure difference in the right ear, **FIG. 11C** shows the admittance of the right ear, **FIG. 11D** shows the pressure difference in the left ear, and **FIG. 11E** shows the admittance of the left ear. The horizontal axis of each graph represents time.

When swallowing is performed, the Eustachian tube briefly opens due to the momentary movement of muscles, which allows outside air to enter the middle ear. Unlike the Valsalva maneuver, which forcefully pushes air into the middle ear through the Eustachian tube, swallowing allows outside air to naturally flow into the middle ear while the Eustachian tube briefly opens. As a result, the eardrum is not fully recovered, and the recovery rate varies from about 50% to 60%, depending on the individual. When the admittance of a subject performing a swallowing action is measured, a maximum admittance value is higher than that observed during the Valsalva maneuver.

As shown in **FIG. 12,** when pressure equalization between the external ear and the inner ear is achieved through swallowing, changes 1210, *i.e.,* a continuous increase, of the admittance prior to the pressure equalization point are followed by changes 1220, *i.e.,* a continuous decrease, of the admittance after the pressure equalization point.

According to the prior art, when the measured admittance value is greater than a predetermined threshold value before the beginning of treatment, it is determined that pressure equalization has occurred, and the Chamber Pressure, which is the current pressure of the chamber 10, is assigned to the variable Middle ear pressure. When the obtained admittance value is lower than the threshold value, it is determined that pressure equalization has not been achieved and the process returns to the beginning of Process 2 without further action. After the initial acoustic admittance is calculated based on 1,024 samples, signals obtained from the MCU module 30 are overlapped by 25%, which is repeated until Process 1 is completed.

According to the prior art using a threshold value, the admittance of the patient's eardrum is measured once before hyperbaric oxygen therapy, and about 40% of the individual's maximum admittance value is set as the threshold value. When the measured admittance is higher than the threshold value, it is determined that pressure equalization has occurred.

However, according to the prior art, the admittance of the patient must be measured before entering the chamber 10 and the maximum and minimum values must be manually input. Also, when a high admittance value caused by noise is measured, the system may incorrectly determine that pressure equalization has occurred even if it has not. Moreover, as the pressure in the chamber 10 increases, the overall admittance values also increase, which reveals a limitation of the prior art using a threshold value.

According to an embodiment, the automatic pressure control system can determine whether pressure equalization has occurred based on a pattern of change in measured admittance.

Referring to **FIG. 10** and **FIG. 12,** a pattern of measured admittance values resulting from the Valsalva maneuver or swallowing shows that when pressure equalization is achieved, there is a continuous upward increase or downward decrease in admittance over a certain period.

According to an embodiment, the automatic pressure control system may define a window on the graph showing a pattern of change in admittance and extract several points (*e.g.*, 6 points) from the initial value to the final value within the window to determine whether the admittance has continuously increased or decreased (Condition 1).

According to an embodiment, When the admittance has continuously increased, the automatic pressure control system may check whether the last point, which is the greatest value within the window, is equal to or greater than a predetermined multiple of the first point (*e.g.*, first point × 1.1 < last point) (Condition 2).

According to an embodiment, when the admittance has continuously decreased, the automatic pressure control system may check whether the first point, which is the greatest value within the window, is equal to or greater than a predetermined multiple of the last point (*e.g.*, last point × 1.1 < first point) (Condition 2).

According to an embodiment, the automatic pressure control system may determine that pressure equalization has occurred only when both Conditions 1 and 2 are satisfied. The automatic pressure control system exhibits robustness to noise even when the admittance value spikes upwards or downwards due to large noise by being configured to simultaneously satisfy both Conditions 1 and 2.

According to an embodiment, the automatic pressure control system may calculate an admittance value by overlapping a portion of received sound wave data.

According to the prior art, the speaker 222 transmits sound waves to the eardrum, and the reflected sound waves are received by the microphone 223. The microphone 223 receives 7,232 data samples per second (7,232 Hz), and by collecting 1,024 of these samples and performing an FFT (Fast Fourier Transform) to convert them into a frequency domain representation, a single data point (admittance) at 226 Hz can be obtained.

According to the prior art, 1,024 of the incoming 7,232 sound wave data samples per second are accumulated and used to calculate an admittance value. Thus, about 7 admittance values per second can be obtained.

According to an embodiment, the automatic pressure control system first calculates an admittance value using accumulated 1,024 sound wave data samples and then discards only a portion (*e.g.*, 100 data samples) and replaces them with 100 new sound wave data samples to form a new set of 1,024 sound wave data samples. Thereafter, the automatic pressure control system obtains an admittance value. Accordingly, after a first admittance value is obtained by accumulating the initial 1,024 sound wave data samples, an admittance value can be obtained every time 100 new sound wave data samples are acquired, and, thus, more admittance values can be obtained. For example, by applying a 90% overlap to the sound wave data, about 70 admittance values per second can be obtained.

Accordingly, by maintaining the accumulated sound wave data and updating only a portion thereof, the automatic pressure control system may improve the real-time responsiveness and increases the number of admittance values and thus enhance the accuracy.

According to an embodiment, the automatic pressure control system may obtain sound wave data and admittance values using the microphone 223 as shown in the following example.

In a right microphone, 12-bit data is bit-shifted 2 bits to the left to form an 8-bit value containing the lower 6 bits and a delimiter bit 00, which is transmitted to the UART unit for communication with the processor 40, and the 12-bit data is bit-shifted 6 bits to the right and 2 bits to the left (equivalent to a total of 4-bit right shift) to form an 8-bit value containing the upper 6 bits and a delimiter bit 01, which is transmitted to the UART unit. Likewise, in a left microphone, 8-bit values containing delimiter bits 10 and 11, respectively, are transmitted to the UART unit.

When a data collection unit of the UART unit receives a continuous sequence of delimiter bits 00, 01, 10 and 11, it completes the collection of Mic Raw data by multiplying the upper 6-bit data by 64 (equivalent to a 6-bit left shift) and adding the lower 6-bit data, excluding the delimiter bits 00 and 01.

The UART unit may filter each data stream using a Point by Point IIR filter. Based on the filter characteristics (second-order IIR filter, Fs = 7,232, Q = 50), forward coefficients are 0.00195965, 0 and -0.00195965 and inverse coefficients are 1, -1.95773 and 0.996081, and 1,024 IIR-filtered microphone data samples are collected, followed by an FFT. Among the 1,024 FFT results, data in the 32nd index (31st if indexing from 0) corresponds to 226-Hz data.

When performing an FFT of N = 1,024 on 7,232-Hz data, the resolution becomes 7,232/1,024 = 7.0625 Hz. Since $7.0625 \times 32 = 226$ Hz, the 32nd index of the 1,024 FFT results corresponds to 226-Hz signal in the sound wave data from the microphone 223.

The window size can be set from 0 to 1,024. For example, if it is set to 100, 100 of 1,024 data samples are discarded and other 100 data samples are collected, followed by an FFT. Given the sampling rate of 7,232 Hz, about 72 data points per second can be obtained.

Since an acoustic admittance is a speaker power or microphone power, the admittance can be obtained using the speaker power of 261,793.72 and the obtained 226-Hz microphone power.

According to an embodiment, the automatic pressure control system may detect pressure equalization based on the obtained admittance value.

For example, when the admittance values at indices 0, 19, 39, 59, 79, 99 and 119 within selected 120 windows increase sequentially, the automatic pressure control system may determine that pressure equalization has occurred when the following Conditions 1 and 2 are satisfied. Further, the current pressure in the chamber 10 can be estimated as the patient's inner ear pressure.

ABT_L[0] < ABT_L[19] & ABT_L[19] < ABT_L[39] & ABT_L[39] < ABT_L[59] & ABT_L[59] < ABT_L[79] & ABT_L[79] < ABT_L[99] & ABT_L[99] < ABT_L[119]    Condition 1:

Condition 2: ABT_L[0] * 1.1 < ABT_L[119]

For example, when the admittance values at indices 0, 19, 39, 59, 79, 99 and 119 within selected 120 windows decrease sequentially, the automatic pressure control system may also determine that pressure equalization has occurred when the following Conditions 1 and 2 are satisfied. Further, the current pressure in the chamber 10 can be estimated as the patient's inner ear pressure.

ABT_L[0] > ABT_L[19] & ABT_L[19] > ABT_L[39] & ABT_L[39] > ABT_L[59] & ABT_L[59] > ABT_L
[79] & ABT_L[79] > ABT_L[99] & ABT_L[99] > ABT_L[119]    Condition 1:


Condition 2: ABT_L[119] * 1.1 < ABT_L[0]


According to an embodiment, the automatic pressure control system may pause pressurization when the pressure difference between the estimated inner ear pressure of the patient and the pressure in the chamber 10 is equal to or greater than 0.06 ata. In an embodiment, if pressure relief has not been achieved within 15 seconds after pausing, the automatic pressure control system may slowly initiate depressurization.

According to an embodiment, if pressure equalization resulted from depressurization is detected or the pressure difference between the estimated inner ear pressure of the patient and the pressure in the chamber 10 is smaller than 0.02 ata, the automatic pressure control system may stop pausing and maintain the current pressure for 7 seconds and then may resume pressurization according to a pressure table.

According to an embodiment, during a treatment standby process before treatment begin, the automatic pressure control system may obtain the pressure of the chamber 10 through the pressure sensor 70. When the treatment starts and Process 1 is performed, the standby process may be ended. The measured pressure of the chamber 10 is input as an initial value of SP, which is generally 1 ata. During treatment, the Chamber Pressure, which is the current pressure of the chamber 10, is obtained through the pressure sensor 70 and assigned to the process variable PV for use by the processor 40. The pressure difference between the external ear and the middle ear, referred to as Difference Pressure (DP), can be calculated based on the middle ear pressure estimated using the current pressure PV and by performing Process 2.

Hold Flag becomes true when the DP value exceeds 0.04 and means that pressure is being maintained by not changing the SP. The initial value of the Hold Flag is false. According to an embodiment, if the Hold Flag is false, which means the state of being pressurized, the automatic pressure control system compares the DP value with 0.04 to determine whether to maintain or increase the pressure. According to an embodiment, if the Hold Flag is true because the DP, which is the pressure difference between the external ear and the middle ear, already exceeds 0.04, the automatic pressure control system compares the DP value with 0.02. Thereafter, if it is smaller than 0.02, the automatic pressure control system determines that pressure imbalance has been resolved. Then, the Hold Flag is set to false and the pressure can be increased by increasing the SP value by 0.002.

According to an embodiment, the automatic pressure control system may obtain an MV using a PID controller based on the SP and PV values determined from the previous calculation results, and then output the MV to control a proportional valve. Therefore, it is possible to regulate the pressure in the chamber 10.

According to an embodiment, when the PV, which is the actual pressure in the chamber 10, is equal to or greater than a desired pressure necessary for treatment, the automatic pressure control system determines that pressurization has been completed, transmits a stop command to the MCU module 30, and terminates the algorithm. This algorithm is applied to both the right and left ears independently, and even if pressure equalization is not achieved in one of the ears, the algorithm continues to be applied.

According to an embodiment, after the patient is positioned inside the chamber 10, the automatic pressure control system may adjust the pressure in the chamber 10 while measuring an admittance. The hyperbaric oxygen chamber operates automatically to increase the pressure at a rate of 0.002 ata/sec. In this case, the patient is spontaneously subjected to continuous pressure equalization. When the pressure in the chamber 10 reaches 1.1 ata, the automatic pressure control system may stop performing any actions that can cause pressure equalization in the patient, and may maintain pressure imbalance. Once it is confirmed that the pressure in the chamber 10 is maintained with a pressure difference of at least 0.04 ata between the middle ear and the external ear by maintaining the pressure imbalance, the patient is subjected to pressure equalization. The above-described process is repeated when the pressure in the chamber 10 reaches 1.2 ata. Once the pressure in the chamber 10 reaches 1.3 ata, the automatic pressure control system may terminate the measurement and return the pressure to the atmospheric pressure.

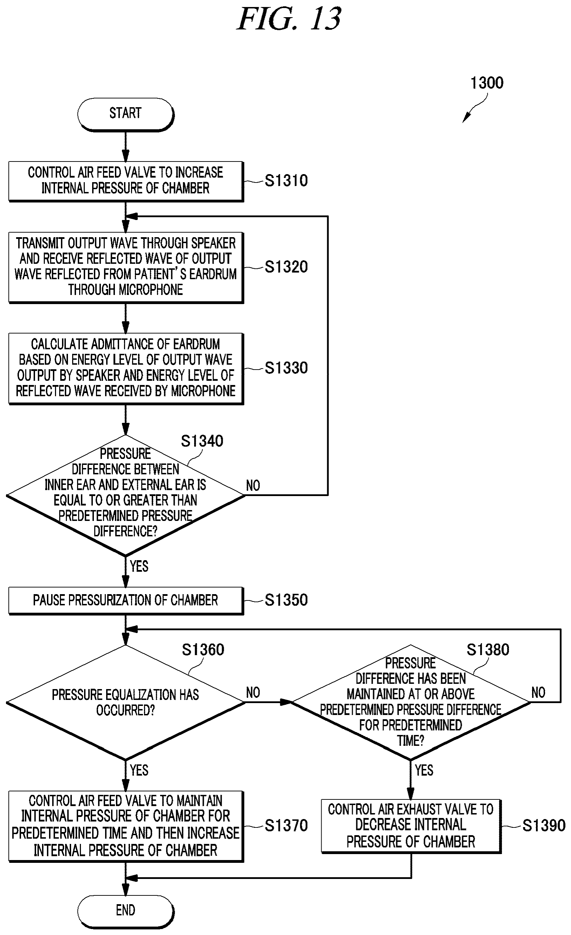

**FIG. 13** is a flowchart 1300 showing an automatic pressure control method of a hyperbaric oxygen chamber for detecting pressure equalization between the external ear and the middle ear according to an embodiment of the present disclosure.

Referring to **FIG. 13,** in a step 1310, the automatic pressure control system of the hyperbaric oxygen chamber according to an embodiment may control the air feed valve 50 to increase the internal pressure of the chamber 10. In an embodiment, the automatic pressure control system of the hyperbaric oxygen chamber may control the air feed valve 50 to increase the internal pressure of the chamber 10 according to a stored treatment profile based on the initiation of hyperbaric oxygen therapy.

According to an embodiment, in a step 1320, the automatic pressure control system of the hyperbaric oxygen chamber may transmit an output wave through the speaker 222 and receive a reflected wave of the output wave reflected from the patient's eardrum through the microphone 223.

According to an embodiment, in a step 1330, the automatic pressure control system of the hyperbaric oxygen chamber may calculate an admittance of the eardrum based on an energy level of the output wave output by the speaker 222 and an energy level of the reflected wave received by the microphone 223.

Herein, the steps 1320 and 1330 may be continuously performed from the start to the end of the hyperbaric oxygen therapy. For example, the steps 1320 and 1330 may be performed at a predetermined operational cycle.

According to an embodiment, in a step 1340, the automatic pressure control system of the hyperbaric oxygen chamber may determine whether the pressure difference between the inner ear and the external ear is equal to or greater than a predetermined pressure difference. For example, the predetermined pressure difference may be 0.06 ata.

According to an embodiment, when the pressure difference between the inner ear and the external ear is equal to or greater than the predetermined pressure difference (step 1340-Yes), the automatic pressure control system of the hyperbaric oxygen chamber may pause the pressurization of the chamber 10 in a step 1350.

According to an embodiment, when the pressure difference between the inner ear and the external ear is smaller than the predetermined pressure difference (step 1340-No), the automatic pressure control system of the hyperbaric oxygen chamber may continue to increase the internal pressure of the chamber 10 according to the stored treatment profile.

According to an embodiment, in a step 1360, the automatic pressure control system of the hyperbaric oxygen chamber may determine whether pressure equalization has occurred based on changes in the calculated admittance. According to an embodiment, in the step 1360, the automatic pressure control system of the hyperbaric oxygen chamber may determine whether pressure equalization has occurred based on accumulated changes in the eardrum's admittance calculated in the steps 1320 and 1330.

According to an embodiment, when it is determined that pressure equalization has occurred (step 1360-Yes), the automatic pressure control system of the hyperbaric oxygen chamber may control the air feed valve 50 to maintain the internal pressure of the chamber 10 for a predetermined time and then increase the internal pressure of the chamber 10 in a step 1370. Herein, the predetermined time may be, for example, 7 seconds. In an embodiment, the automatic pressure control system of the hyperbaric oxygen chamber may continue to increase the internal pressure of the chamber 10 according to the stored treatment profile.

According to an embodiment, when it is determined that pressure equalization has not been achieved (step 1360-No), the automatic pressure control system of the hyperbaric oxygen chamber may determine whether the pressure difference is maintained at or above the predetermined pressure difference for a predetermined time in a step 1380.

According to an embodiment, when it is determined that the pressure difference has been maintained at or above the predetermined pressure difference for the predetermined time (step 1380-Yes), the automatic pressure control system of the hyperbaric oxygen chamber may control the air exhaust valve 60 to decrease the internal pressure of the chamber 10 in a step 1390.

According to an embodiment, when it is determined that the pressure difference has not been maintained at or above the predetermined pressure difference for the predetermined time (step 1380-No), the automatic pressure control system of the hyperbaric oxygen chamber may determine whether pressure equalization has occurred in a state where the pressurization of the chamber 10 is paused, in the step 1360.

In the automatic pressure control system of the hyperbaric oxygen chamber for detecting the pressure equilibrium state between the outer ear and the middle ear as described above, the automatic pressure control method of the hyperbaric oxygen chamber for detecting the pressure equilibrium state between the outer ear and the middle ear may also be implemented in the form of a computer-readable recording medium storing a computer program or computer-executable instructions executed by a computer. In addition, the automatic pressure control method for detecting a pressure equilibrium state between the outer ear and the middle ear in a hyperbaric oxygen chamber as described above may also be implemented in the form of a computer program stored in a computer-readable recording medium executed by a computer.

A computer-readable recording medium may be any available medium that can be accessed by a computer, and includes both volatile and non-volatile media, as well as removable and non-removable media. Furthermore, a computer-readable recording medium may include computer storage media. Computer storage media include both volatile and non-volatile, removable and non-removable media implemented in any method or technology for storage of information such as computer-readable instructions, data structures, program modules, or other data.

The functions realized by the components described in this specification may be implemented in processing circuitry including a general-purpose processor programmed to perform the described functions, a special-purpose processor, an integrated circuit, application specific integrated circuits (ASICs), a central processing unit (CPU), circuitry, and/or combinations thereof. The processor may include transistors or other circuitry and may be considered as circuitry or processing circuitry. The processor may be a programmed processor that executes a program stored in memory.

In this specification, the terms circuit, part, unit, and means refer to hardware programmed or operated to perform the described functions. Such hardware may be any hardware disclosed in this specification or any hardware known to be programmed or operated to perform the described functions.

If such hardware is a processor regarded as a circuit type, the circuit, part, means, or unit refers to a combination of hardware and software used to configure the hardware and/or processor.

The foregoing description of the present disclosure is for illustrative purposes, and those skilled in the art to which the present disclosure pertains will understand that various modifications can be made without departing from the technical spirit or essential features of the present disclosure. Therefore, it should be understood that the above-described embodiments are exemplary in all respects and not restrictive. For example, each component described as a single entity may be implemented in a distributed manner, and likewise, components described as distributed may be implemented in a combined form.

The scope of the present disclosure is defined not by the above detailed description but by the claims described below, and all modifications or altered forms derived from the meaning and scope of the claims and their equivalents should be construed as being included within the scope of the present disclosure.

**Claims**

1. An automatic pressure control system of a hyperbaric oxygen chamber for detecting pressure equalization between the external ear and the middle ear, comprising:

   a chamber having an internal space in which a patient can be positioned;
   a probe configured to be inserted into the patient's ear and having a speaker configured to output sound waves and a microphone configured to receive sound waves;
   an MCU module electrically connected to the probe;
   an air feed valve configured to open or close an air feed line connected to the chamber;
   an air exhaust valve configured to open or close an air exhaust line connected to the chamber; and
   at least one processor,
   wherein the MCU module is configured to transmit an output wave through the speaker and receives a reflected wave of the output wave reflected from the patient's eardrum through the microphone,
   wherein the at least one processor is configured to:

      calculate an admittance of the eardrum based on an energy level of the output wave output by the speaker and an energy level of the reflected wave received by the microphone,
      determine whether the pressure equalization has occurred based on changes in the calculated admittance, and
      control the air feed valve based on the determination of whether the pressure equalization has occurred.

2. The automatic pressure control system of Claim 1,
   wherein the at least one processor is configured:
   in determining whether the pressure equilibrium state has occurred, to determine that the pressure equalization has occurred when the admittance continues to increase or decrease within a window of a predetermined size.

3. The automatic pressure control system of Claim 2,
   wherein the window includes a predetermined number of admittance values.

4. The automatic pressure control system of Claim 2,
   wherein the at least one processor is configured:
   in determining whether the pressure equilibrium state has occurred, to determine that the pressure equalization has occurred when the admittance continues to increase within the window and a last admittance value within the window is equal to or greater than a predetermined multiple of a first admittance value.

5. The automatic pressure control system of Claim 2,
   wherein the at least one processor is configured:
   in determining whether the pressure equilibrium state has occurred, to determine that the pressure equalization has occurred when the admittance continues to decrease within the window and a first admittance value within the window

is equal to or greater than a predetermined multiple of a last admittance value.

6. The automatic pressure control system of Claim 1,
   wherein the at least one processor is configured to:
   calculate the admittance by accumulating a predetermined amount of time-dependent data based on an energy level of the output wave and an energy level of the reflected wave, overlapping a portion of the predetermined accumulation amount of data, and updating only the remaining portion.

7. The automatic pressure control system of Claim 1,
   wherein the at least one processor is configured to:

   control the air feed valve to increase an internal pressure of the chamber, and
   pause pressurization of the chamber based on the calculated admittance when a pressure difference between the inner ear and the external ear is equal to or greater than a predetermined pressure difference.

8. The automatic pressure control system of Claim 7,
   wherein the at least one processor is configured to:
   control the air exhaust valve to decrease the internal pressure of the chamber when the pressure difference between the inner ear and the external ear is maintained at or above the predetermined pressure difference for a predetermined time in a state where the pressurization of the chamber is paused.

9. The automatic pressure control system of Claim 1,
   wherein the at least one processor is configured to:
   in controlling the air feed valve, when it is determined that the pressure equalization has occurred, control the air feed valve to maintain an internal pressure of the chamber for a predetermined time and then increase the internal pressure of the chamber.

10. An automatic pressure control method of a hyperbaric oxygen chamber for detecting pressure equalization between the external ear and the middle ear, comprising:

    transmitting an output wave through a speaker and receiving a reflected wave of the output wave reflected from a patient's eardrum through a microphone, wherein the speaker and the microphone included in a probe configured to be inserted into the patient's ear;
    calculating an admittance of the eardrum based on an energy level of the output wave output by the speaker and an energy level of the reflected wave received by the microphone;
    determining whether the pressure equalization has occurred based on changes in the calculated admittance; and
    controlling an air feed valve, which is configured to open or close an air feed line connected to a chamber, based on the determination of whether the pressure equalization has occurred.

11. The automatic pressure control method of Claim 10,
    wherein the determining whether the pressure equalization has occurred includes determining that the pressure equalization has occurred when the admittance continues to increase or decrease within a window of a predetermined size.

12. The automatic pressure control method of Claim 11,
    wherein the determining whether the pressure equalization has occurred includes determining that the pressure equalization has occurred when the admittance continues to increase within the window and a last admittance value within the window is equal to or greater than a predetermined multiple of a first admittance value.

13. The automatic pressure control method of Claim 11,
    wherein the determining whether the pressure equalization has occurred includes determining that the pressure equalization has occurred when the admittance continues to increase within the window and a last admittance value within the window is equal to or greater than a predetermined multiple of a first admittance value.

14. The automatic pressure control method of Claim 11,
    wherein the determining whether the pressure equalization has occurred includes determining that the pressure equalization has occurred when the admittance continues to decrease within the window and a first admittance value within the window is equal to or greater than a predetermined multiple of a last admittance value.

**15.** The automatic pressure control method of Claim 10:
wherein in the calculating an admittance, calculating the admittance by accumulating a predetermined amount of time-dependent data based on an energy level of the output wave and an energy level of the reflected wave, a portion of the predetermined accumulation amount of data is overlapped, and only the remaining portion is updated.

*FIG. 1*

*FIG. 2*

# FIG. 3

# FIG. 4

external ear

222

external ear

ear drum

224

middle ear

223

*FIG. 5*

## FIG. 6

301

## FIG. 7

302

AVCC3.3

From microphone →

AR3
1M_1608_1/16W_J

AC2
100nF_1608_16V_J

AR4
1M_1608_1/16W_J

AGND

AU1C
OPA4336EA

12
11

10

To MCU ADC →

Signal gain 25

AR5
360k_1608_1/16W_J

AR6
15k_1608_1/16W_J

AC3
100nF_1608_16V_J

AGND

EP 4 736 829 A1

EP 4 736 829 A1

# FIG. 8A

# FIG. 8B

④

**Left Ear Inner Pressure**

**Right Ear Inner Pressure**

⑤

**Left DP**

**Right DP**

EP 4 736 829 A1

*FIG. 8C*

⑥

**Left Ear Admittance**

**Right Ear Admittance**

EP 4 736 829 A1

## FIG. 9A

## FIG. 9B

Right Ear Pressure Difference

*FIG. 9C*

Right Ear Admittance

## FIG. 9D

Left Ear Pressure Difference — Pressure(ata) vs Time(sec)

EP 4 736 829 A1

## FIG. 9E

Left Ear Admittance

EP 4 736 829 A1

## FIG. 10

# FIG. 11A

*FIG. 11B*

Right Ear Pressure Difference

EP 4 736 829 A1

## FIG. 11C

Right Ear Admittance

## FIG. 11D

Left Ear Pressure Difference

## FIG. 11E

Left Ear Admittance

# FIG. 12

Right Ear Admittance

1210    1220

## FIG. 13

1300

START

CONTROL AIR FEED VALVE TO INCREASE INTERNAL PRESSURE OF CHAMBER — S1310

TRANSMIT OUTPUT WAVE THROUGH SPEAKER AND RECEIVE REFLECTED WAVE OF OUTPUT WAVE REFLECTED FROM PATIENT'S EARDRUM THROUGH MICROPHONE — S1320

CALCULATE ADMITTANCE OF EARDRUM BASED ON ENERGY LEVEL OF OUTPUT WAVE OUTPUT BY SPEAKER AND ENERGY LEVEL OF REFLECTED WAVE RECEIVED BY MICROPHONE — S1330

S1340
PRESSURE DIFFERENCE BETWEEN INNER EAR AND EXTERNAL EAR IS EQUAL TO OR GREATER THAN PREDETERMINED PRESSURE DIFFERENCE? — NO

YES

PAUSE PRESSURIZATION OF CHAMBER — S1350

S1360
PRESSURE EQUALIZATION HAS OCCURRED? — NO

S1380
PRESSURE DIFFERENCE HAS BEEN MAINTAINED AT OR ABOVE PREDETERMINED PRESSURE DIFFERENCE FOR PREDETERMINED TIME? — NO

YES

YES

CONTROL AIR FEED VALVE TO MAINTAIN INTERNAL PRESSURE OF CHAMBER FOR PREDETERMINED TIME AND THEN INCREASE INTERNAL PRESSURE OF CHAMBER — S1370

CONTROL AIR EXHAUST VALVE TO DECREASE INTERNAL PRESSURE OF CHAMBER — S1390

END

TRANSLATION

## INTERNATIONAL SEARCH REPORT

| International application No. |
|---|
| **PCT/KR2024/012675** |

### A. CLASSIFICATION OF SUBJECT MATTER

**A61G 10/02**(2006.01)i; **A61G 10/00**(2006.01)i; **A61B 5/03**(2006.01)i; **H04R 1/08**(2006.01)i; **H04R 1/10**(2006.01)i; **H04R 1/32**(2006.01)i

According to International Patent Classification (IPC) or to both national classification and IPC

### B. FIELDS SEARCHED

Minimum documentation searched (classification system followed by classification symbols)

A61G 10/02(2006.01); A61G 10/00(2006.01); H04R 1/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 외이(outer ear), 중이(middle ear), 압력평형(pressure equilibrium), 어드미턴스 (admittance), 고압산소탱크(high-pressure oxygen tank), 밸브(valve)

### C. DOCUMENTS CONSIDERED TO BE RELEVANT

| Category* | Citation of document, with indication, where appropriate, of the relevant passages | Relevant to claim No. |
|---|---|---|
| X | KR 10-2022-0084673 A (IBEX MEDICAL SYSTEMS CO., LTD.) 21 June 2022 (2022-06-21) claims 1-5; paragraphs [0001], [0016]; figures 1, 3, 4 | 1,7,10 |
| A | | 2-6,8-9,11-15 |
| A | KR 10-2022-0084675 A (IBEX MEDICAL SYSTEMS CO., LTD.) 21 June 2022 (2022-06-21) claims 1, 2; paragraphs [0019], [0020]; figures 1, 3, 4 | 1-15 |
| A | KR 10-1685691 B1 (IBEX MEDICAL SYSTEMS CO., LTD. et al.) 20 December 2016 (2016-12-20) claim 5; figures 1, 4, 5 | 1-15 |
| A | KR 10-2022-0084676 A (IBEX MEDICAL SYSTEMS CO., LTD.) 21 June 2022 (2022-06-21) claims 1-3; paragraphs [0019], [0020]; figures 1, 2, 4 | 1-15 |
| A | JP 2017-509284 A (BOSE CORP.) 30 March 2017 (2017-03-30) claims 1, 2; figures 1-4 | 1-15 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 May 2025** | **19 May 2025** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

TRANSLATION

**INTERNATIONAL SEARCH REPORT**
**Information on patent family members**

International application No.

**PCT/KR2024/012675**

| Patent document cited in search report | | | Publication date (day/month/year) | Patent family member(s) | | | Publication date (day/month/year) |
|---|---|---|---|---|---|---|---|
| KR | 10-2022-0084673 | A | 21 June 2022 | KR | 10-2543402 | B1 | 14 June 2023 |
| KR | 10-2022-0084675 | A | 21 June 2022 | KR | 10-2543403 | B1 | 14 June 2023 |
| KR | 10-1685691 | B1 | 20 December 2016 | | None | | |
| KR | 10-2022-0084676 | A | 21 June 2022 | KR | 10-2543412 | B1 | 14 June 2023 |
| JP | 2017-509284 | A | 30 March 2017 | CN | 106105256 | A | 09 November 2016 |
| | | | | CN | 106105256 | B | 26 April 2019 |
| | | | | EP | 3117629 | A1 | 18 January 2017 |
| | | | | EP | 3117629 | B1 | 03 October 2018 |
| | | | | JP | 6313480 | B2 | 18 April 2018 |
| | | | | US | 2015-0264467 | A1 | 17 September 2015 |
| | | | | US | 9301040 | B2 | 29 March 2016 |
| | | | | WO | 2015-138829 | A1 | 17 September 2015 |

Form PCT/ISA/210 (patent family annex) (July 2022)